# EUROPEAN PATENT SPECIFICATION

(11) **EP 0 875 247 B1**
(45) Date of publication and mention of the grant of the patent: **28.05.2003**
(21) Application number: 98201255.1
(22) Date of filing: 20.04.1998
(51) Int. Cl.: A61K 31/55, A61K 31/34, A61P 9/10

(54) **Method for controlling the plasma level of lipoproteins**
Blutlipoproteinspiegel regulierendes Präparat
Médicament régulateur du niveau des lipoprotéines du sang

(30) Priority: 18.04.1997 EP 97201139
(43) Date of publication of application: 04.11.1998
(73) Proprietor: NEDERLANDSE ORGANISATIE VOOR TOEGEPAST-NATUURWETENSCHAPPELIJK ONDERZOEK TNO, 2628 VK Delft (NL)
(72) Inventor: Princen, Johannes Marinus Gerardus, 2343 HV Oegstgeest (NL); Kooistra, Take, 2318 NH Leiden (NL)
(74) Representative: Prins, Adrianus Willem

(56) References cited:
- EP-A- 0 178 261
- EP-A- 0 402 151
- EP-A- 0 459 432
- WO-A-96/20941
- US-A- 5 302 590
- R. FELISTE ET AL: "Protective effect of BN 52021, a specific antagonist of platelet-activating factor (PAF-acether) against diet-induced cholesteryl ester deposition in rabbit aorta" ATHEROSCLEROSIS, vol. 78, 1989, IRELAND, pages 151-158, XP002045405
- M. KOLTAI ET AL: "Platelet Activating Factor (PAF): A review of its Effects, Antagonists and Possible Future Clinical Implications (Part I)" DRUGS, vol. 42, no. 1, 1991, pages 9-29, XP000576884
- WHITTAKER M: "PAF RECEPTOR ANTAGONISTS: RECENT ADVANCES" CURRENT OPINION IN THERAPEUTIC PATENTS, vol. 2, no. 5, 1 May 1992, pages 583-623, XP000576557

## Description

### Field of the invention

The present invention relates to the use of active substances for preparing a medicament controlling the plasma level of the lipoproteins lipoprotein(a) (Lp(a)) and high-density lipoprotein (HDL) by decreasing the synthesis of apolipoprotein(a) (apo(a)) and by increasing the synthesis of apolipoprotein A_{I} (apo A_{I}). In particular, the invention relates to the novel use of antagonists of platelet activating factor (PAF-antagonists) and of structurally related compounds, which do not necessarily possess PAF-antagonistic activity, for preparing such medicaments.

### Background

Apolipoprotein A-I (apo A-I) is the major protein constituent of plasma high density lipoprotein (HDL) (1). In mammals, the protein is mainly synthesized in the liver and the small intestine (1,2). Decreased plasma levels of HDL cholesterol are associated with an accelerated development of atherosclerotic lesions, which is one of the main causes of coronary artery disease (3-5). The plasma level of apo A-I has been reported to be even more discriminatory in determining the risk of cardiovascular disease than the cholesterol concentration of HDL (6,7).

Several drugs (e.g. fibrates and statins, summarized in refs. 5 and 12) are currently in use to lower plasma lipid levels, which secondarily increase plasma HDL levels. However, their HDL increasing effects are small in general and most of them, perhaps with the exception of gemfibrozil, appear to act indirectly, i.e. no direct effect on apo A-I synthesis is found (5,12). Recently, a series of urea-type compounds and thiazolo-[3,2-c]pyrimidine-5,7-diones have been claimed as HDL-elevators (see ref 95,96 in 12).

A high plasma level of lipoprotein(a) (Lp(a)) is positively associated with the development of coronary heart disease and cerebrovascular disease in men and women, especially when plasma levels exceed 0,20-0,30 g/l and when LDL levels are concomitantly increased (13-15). In Lp(a) the apoprotein (a), which is synthesized in the liver, is covalently bound to an LDL particle. This binding is thought to take place extracellularly at the cell surface (12). In vivo turnover studies in humans indicated that Lp(a) levels are mainly influenced by Lp(a) and apo(a) production rates and not by Lp(a) clearance rates, emphasizing the importance of apo(a) synthesis for plasma Lp(a) levels (16,17).

No satisfactory pharmacological treatment for raised Lp(a) levels exists currently. Of the established hypolipidemic drugs only nicotinic acid and perhaps some fibrates, as well as LDL apheresis treatment lower Lp(a) levels, but these therapies are inadequate in terms of efficacy, specificity and palatability (12,14,15).

Apo B-100 is the sole protein of LDL. Increased levels of LDL-cholesterol and apo B-100 in blood are strongly associated with the development of atherosclerosis and the incidence of coronary heart disease (8-11).

### Summary of the invention

It was found that administration of certain compounds which have PAF (platelet activating factor) antagonistic activity and structurally related compounds, which may or may not have such activity, results in a reduced synthesis of apolipoprotein(a) (apo(a)) and/or, independently, in an enhanced synthesis of apolipoprotein A_{I} (apo A_{I}).

### Detailed description

In a first aspect, the invention pertains to the use of compounds active as PAF-antagonists and structurally related compounds which need not be active as PAF-antagonists in the prevention and treatment of atherogenic conditions in mammals including man, by decreasing lipoprotein (a) (Lp(a)) levels in plasma. In a second aspect, the invention pertains to such compounds in the prevention and treatment of atherogenic conditions by increasing HDL levels in plasma. It should be understood that, according to the invention, the term "structurally related" refers to compounds which have structural similarities, especially skeletal identity - as defined in the appending claims - with the PAF-antagonists, but which need not be active as a PAF-antagonist.

Suitable PAF-antagonists are described, for example, by Hwang in *J. Lipid Mediators,* **2** (1990) 123-158 (ref. 18). Examples include various benzo- and thieno-diazepines, bis(trimethoxyphenyl)- or bis(dimethoxyphenyl)-dioxolanes and -tetrahydrofurans, especially trans-2,5-bis(3,4,5-trimethoxyphenyl)tetrahydrofuran (L-652,731) and cis- and trans-2,4-bis(3,4,5-trimethoxyphenyl)dioxalane, and various phospholipid analogues such as *O*-cis-5-(octadecylcarbamoyloxymethyl)-2-tetrahydrofurylmethyl *O*-(2-quinolinio-ethyl) hydrogen phosphate (SRI 63-441). These and other PAF-antagonists are also described by Saunders and Handley (ref. 19) and by Weber and Heuer (ref. 20).

The only effective pharmacological treatment known thus far for reducing Lp(a) levels consists in administering nicotinic acid or its derivatives, either in combination with neomycine (3 g/day and 2 g/day respectively, see ref. 23) or alone (4 g/day, see ref. 24). Reductions of plasma levels of lp(a) were in the order of 45% and 38%, respectively.

The benzo- and thieno(di)azepines constitute an advantageous group of compounds for inhibiting apo(a) synthesis according to the invention. The benzo(di)azepines are also useful according to the invention for promoting apo A-I synthesis. Most of these compounds are known per se. Examples of specific (di)azepines and their synthesis are described in US patent 5,302,590 and in WO 96/20941.

The known effects of the benzodiazepines virtually all result from the actions of the drugs on the central nervous system. The most prominent of these effects are sedation, hypnosis, decreased anxiety, muscle relaxation, anterograde amnesia, and anticonvulsant activity. Only two effects of the drugs appear to result from actions on peripheral tissues: coronary vasodilatation, seen after intravenous administration of therapeutic doses of certain benzodiazepines, and neuromuscular blockade, seen only with very high doses (cf. ref. 21).

It was reported in 1984 that psychotropic triazolobenzodiazepines can inhibit the aggregation of platelets induced by platelet activating factor (22). Platelet-activating factor (PAF) is a potent inflammatory mediator with a wide variety of biological activities, such as induction of platelet and neutrophil aggregation, bronchoconstriction, hypotension and an increase in vascular permeability (19). Thus, PAF can induce a profile of biological effects which can mimic many of the major features of asthma. PAF-antagonists can be used in the management or prophylactic control of asthma or other inflammatory and allergic conditions (19,20). Many research groups were successful in separating CNS activity from PAF antagonism (20).

The benzo- and thieno-(di)azepines to be used according to the invention are defined in the appending claims. It may be noted that the substituent represented by R^{2'} in the cyclopentane-2-yl-1-thiyl group represented by symbol A may be substituted phenyl. Examples of substituents in this respect include alkyl, alkoxy, halo, hydroxyl, carbamoyl, carboxyl and alkoxycarbonyl and combinations thereof, such as a combination of N-{3-[2-(4-cycloalkyl-2-thiazolyl)ethenyl]phenyl}carbamoyl and carboxyl, ethoxycarbonyl or carbamoyl as described in US 5,302,590.

The diphenyl-tetrahydrofuran and diphenyl-dioxolane derivatives, as well as the tetrahydrofuran phospholipids are another group of suitable compounds according to the invention, and are defined in the claims.

The specificity of the reported effects is underlined by the absence of any effect on apo B-100 and albumin synthesis.

The pharmaceutical compositions to be prepared according to the invention may be formulated in the usual way, e.g. by associating the compounds with a pharmaceutically suitable solid or liquid carrier and optional adjuvants or other active components, stabilisers, colorants, flavourings etc. The composition may be suitable for oral administration (capsule, pill, tablet, gel, powder, sachet, syrup, solution, dispersion etc.) or may be an injectable solution or another administration form (e.g. via suppositories or via plasters). The effective dose is between 0.01 and 30 mg per kg body weight per day, preferably between 0.1 and 10 mg/kg.day. A dose can be administered in a single dosage or in several daily dosages.

### Examples

### Simian hepatocyte isolation and culture

Simian hepatocytes were isolated from livers of both male and female monkeys. The monkeys were 1.5 to 3 years old and were obtained from the National Institute of Public Health and Environmental Protection (RIVM), Bilthoven, The Netherlands.

The monkeys were bred at the RIVM and served as donors for kidneys used in the production of poliomyelitis vaccine at this institute. The isolation procedure was performed as described previously (25). Viability, based on the ability of hepatocytes to exclude trypan blue dye (0.11%) was 66-96%. Total cell yields varied from 0.74 to 2.3 x 10⁹ viable cells. The cells were seeded on culture dishes at a density of 1.5 x 10⁵ viable cells per square cm and were maintained for the first 24 h in 1.5 mL per 10 square cm of Williams E medium supplemented with 10% heat inactivated (30 min at 56°C) fetal calf serum (FCS) (Boehringer Mannheim), 2 mmol/L L-glutamine, 20 mU/mL insulin (135 nmol/L), 50 nmol/L dexamethasone, 100 U/mL penicillin, 100 µg/mL streptomycin and 100 µg/mL kanamycin at 37°C in a 5% CO₂/95% air atmosphere. After 14-16 h the non-adherent cells were washed from the plates, using the same culture medium as described above. Twenty-four hours after seeding, the incubations with the compounds were started in 1 mL of the same culture medium, but with a lower insulin concentration, 10 nmol/L instead of 135 nmol/L. The medium was renewed every 24 h. At the end of each incubation period the medium was collected and centrifuged for 30 seconds in an Eppendorf centrifuge at maximum speed to remove debris and detached cells. The supernatant was frozen in dry ice and stored at -20°C until measurement of apolipoproteins. After the last incubation, the cells were washed three times with cold phosphate-buffered saline (PBS), pH 7.4, and cellular protein was determined. All incubations, control and with the compounds at various concentrations, were performed with medium containing 0.1% (v/v) DMSO. Materials used for the isolation and culturing of the simian hepatocytes were obtained from sources described previously (25).

### Isolation and culture of human hepatocytes

Human hepatocytes were isolated from pieces of livers, obtained from donors which could not be used for transplantation. Hepatocytes were isolated essentially as described previously in detail (26-28) and were cultured as described for simian hepatocytes, with the exception that incubations were started between 24 and 36 h after isolation of the hepatocytes.

### Apo(a), apo A-I and apo B-100 ELISA

Total apo(a) concentrations were determined using the TintElize Lp(a) (Biopool AB, Umeå, Sweden). This ELISA uses polyclonal antibodies to human apo(a) both as catching and as detecting antibodies and detects in this manner free apo(a) as well as lp(a). The antibodies of this kit showed strong immunological cross-reactivity with lp(a) from cynomolgus monkeys with standard-curves parallel to human lp(a), indicating a high level of homology between human and cynomolgus lp(a), in accordance with Makino et al (29) and Azrolan et al (30).

Apo A-1 and apo B-100 concentrations in the medium were measured using a sandwich enzyme-linked immunosorbent assay (ELISA) with polyclonal antibodies to human apo A-I or human apo B-100, respectively, both as catching and detecting antibodies as described previously (31,32). The standard curves for apo A-I and apo B-100 in human and cynomolgus monkey sera and in medium of cultured cynomolgus and human hepatocytes were parallel, indicating that similar epitopes on apo A-I and apo B-100 of the two species are recognized.

Data shown in table 1 are obtained from measurements in media of hepatocytes cultured for 72h with or without the compounds. i.e. culture period from 48h to 72h.

### RNA hybridization

Total RNA was isolated from cynomolgus hepatocytes, cultured for 48h in the presence or absence of the compounds, by the method of Chomczynski & Sacchi (33). After washing the RNA pellets with 70% (v/v) ethanol, RNA samples were dissolved in water. The RNA concentration in each sample was determined spectrophotometrically, assuming that one A₂₆₀ unit corresponds with 40 µg RNA/mL. Equal amounts of total RNA (10 µg) from different incubations were fractionated by electrophoresis in a 0.8% (w/v) agarose gel containing 0.27 M formaldehyde, and transferred to Hybond-N+ (Amersham) in accordance with the manufacturer's instructions and UV cross-linked. Probes, labelling conditions and hybridization were performed as described previously in detail (25).

Apo(a) mRNAs were detected using a kringle IV synthetic double-stranded probe of 75 nucleotides with the sense sequence: GGGAATTCGA ACCTGCCAAG CTTGGTCATC TATGACACCA CACTCGCATA GTCGGACCCC AGAATAAAGC TTGGG, based on the sequence published by McLean et al (34). This probe was labelled by the random primer method according to Megaprime™ DNA labelling systems (Amersham). After hybridization, the blots were washed twice with 2 x SSC/1% SDS (1 x SSC = 0.15 mol/L NaCl/0.015 mol/L sodium citrate, pH 7.0) and twice with 1 x SSC/1% SDS for 30 min at 65°C. The blots were exposed to a Fuji imaging plate type BAS-MP for 1 to 24h. The relative amounts of mRNA were quantified using a phospho-imager (Fuji Fujix BAS 1000) and the computer programs BAS-reader version 2.8 and TINA version 2.08c.

Data shown in table 2 are obtained from measurements using RNA isolated from hepatocytes cultured for 48h with or without the compounds.

**Table 1.**

| Effects of the compounds on synthesis of apo A-I, apo(a) and apo B-100 by cynomolgus monkey and human hepatocytes | | | | |
|---|---|---|---|---|
| Compound | Conc. µM | Apo A-I | Apo(a) % of control ¹ | Apo B-100 |
| Synthesis in cynomolgus (simian) hepatocytes | | | | |
| Azepine 1² | 3 | 152±30 (2) | 85±5 (2) | 117±10 (2) |
| | 10 | 185±20 (6) | 76±19 (6) | 113±20 (6) |
| | 30 | 225±45 (6) | 48±28 (6) | 106±27 (6) |
| | 100 | 367±131 (6) | 34±32 (6) | 95±23 (6) |
| | | | | |
| Azepine 2² | 30 | 178 (1) | no change | not tested |
| | | | | |
| Azepine 3² | 100 | 359±74 (3) | 42±20 (3) | 102±32 (2) |
| | | | | |
| Azepine 4² | 100 | 390±40 (2) | 62±22 (2) | 95±23 (2) |
| | | | | |
| Transdioxolane³ | 30 | 116±7 (3) | 103±9 (3) | 117±23 (3) |
| | 100 | 157±2 (3) | 60±12 (3) | 105±7 (3) |
| | | | | |
| Cisdioxolane⁴ | 100 | 229±79 (2) | 83±2 (3) | 99±21 (3) |
| | | | | |
| L-652,731⁵ | 100 | 134±1 (2) | 66±8 (2) | 76±35 (2) |
| | | | | |
| SRI 63-441⁶ | 100 | 166±6 (2) | 24±1 (2) | 142±32 (2) |
| | | | | |
| BN 52051⁷ | 100 | 144±34 (2) | 101±1 (2) | 113±34 (2) |

| Synthesis in human hepatocytes | | | | |
|---|---|---|---|---|
| Azepine 1² | 3 | 164±61 (2) | 75 (1) | 83±16 (2) |
| | 10 | 283±109 (2) | 48 (1) | 112±3 (2) |
| | 30 | 622±228 (2) | 24 (1) | 109±8 (2) |
| | 100 | 721±212 (2) | 14 (1) | 114±8 (2) |

| | | | | |
|---|---|---|---|---|
| ¹ Values are means ± S.D. or range of duplicate incubations of the number of independent culture experiments indicated in parentheses. | | | | |
| ² Azepine 1 has formula 1, A= CH=CH-S, X=Y=Z=N, R¹=CH₃, R³=R⁴=H Azepine 2 has formula 1, A= CH=CH-S, X=Y=Z=N, R¹=R³=R⁴=H Azepine 3 has formula 1, A= CH=CH-CCl=CH, X=Y=Z=N, R¹=CH₃, R³=CH₃, R⁴=H Azepine 4 has formula 1, A= CH=CH-CH=CH, X=Y=Z=N, R¹=Br, R³=R⁴=H | | | | |
| ³ Trans-2,4-bis(3,4,5-trimethoxyphenyl)dioxolane, commercially available | | | | |
| ⁴ Cis-2,4-bis(3,4,5-trimethoxyphenyl)dioxolane, commercially available | | | | |
| ⁵ Trans-2,5-bis(3,4,5-trimethoxyphenyl)tetrahydrofuran | | | | |
| ⁶ The phospholipid analogue with formula 2, wherein Z is vinylene, R⁵ and R⁶ together form benzo[b], R⁷ is hydrogen, R⁸ is octadecyl and n is 1, | | | | |
| ⁷ Ginkgolide B, commercially available | | | | |

**Table 2.**

| Effect of azepine 1 on mRNA levels of apo A-1 and apo(a) in cynomolgus monkey and human hepatocytes | | | |
|---|---|---|---|
| Compound µM | Conc. % of control ¹ | Apo A-I | Apo(a) |
| mRNA levels relative to rRNA in cynomolgus (simian) hepatocytes | | | |
| azepine 1 | 100 | 229±8 (3) | 56±12 (3) |
| | | | |

| mRNA levels relative to GAPDH in cynomolgus (simian) hepatocytes | | | |
|---|---|---|---|
| azepine 1 | 100 | 147±28 (3) | 39±4 (3) |
| | | | |

| mRNA levels relative to actin in cynomolgus (simian) hepatocytes | | | |
|---|---|---|---|
| azepine 1 | 100 | 328±7 (3) | 86±7 (3) |
| | | | |

| mRNA levels relative to rRNA in human hepatocytes | | | |
|---|---|---|---|
| azepine 1 | 100 | 308 (1) | 67 (1) |
| | | | |

| mRNA levels relative to GAPDH in human hepatocytes | | | |
|---|---|---|---|
| azepine 1 | 100 | 111 (1) | 24 (1) |
| | | | |

| mRNA levels relative to actin in human hepatocytes | | | |
|---|---|---|---|
| azepine 1 | 100 | 203 (1) | 44 (1) |

| | | | |
|---|---|---|---|
| 1 Values are means ± S.D. of the number of independent culture experiments indicated in the parentheses. The increased apo A-I mRNA levels indicate that the increased apo A-I synthesis is regulated at the (post)-transcription level. The decreased apo (a) mRNA levels indicate that the decreased apo (a) synthesis is regulated at the (post)-transcription level. | | | |

### References

1. Eisenberg S: J Lipid Res 1984; 25: 1017-1058.
2. Brewer HB Jr, Fairwell T, LaRue A, Ronan R, Houser A, Bronzert TJ: Biochem Biophys Res Commun 1978; 80: 623-630.
3. Gordon T, Castelli WP, Hjortland MC, Kannel WB, Dawber TR: Am J Med 1977; 62: 707-714.
4. Breslow JL: Familial disorders of high density lipoprotein metabolism, in The Metabolic Basis of Inherited Disease (Scriver CR, Beaudet AL, Sly WS, Valle D, Eds). McGraw-Hill, New York, 1989: pp 1251-1266.
5. Gordon DJ, Rifkind BM: N Engl J Med 1989; 321: 1311-1316.
6. Glueck CJ, Gartside P, Fallat RW, Sielski J, Steiner PM: J. Lab Clin Med 1976; 88: 941-957.
7. Brunzell JD, Sniderman AD, Albers JJ, Kwiterovich PO Jr: Arteriosclerosis 1984; 4: 79-83.
8. Lipid Research Clinics Programme: the Lipid Research Clinics Coronary Prevention Trial Results: II. The relationship of reduction in incidence of coronary heart disease to cholesterol lowering. JAMA 1984; 251: 365-374.
9. Kesaniemi YA, Grundy SM. Arteriosclerosis 1983; 3: 40-46.
10. Teng B, Sniderman AD, Soutar AK, Thompson GR: J Clin Invest 1986; 77: 663-672.
11. Castelli WP, Garrison RJ, Wilson PWF, Abbott RD, Kalousdian S, Kannel WB: JAMA 1986; 256: 2835-2838.
12. Krause BR, Sliskovic DR, Bocan TMA: Emerging therapies in atherosclerosis. Exp Opin Invest Drugs 1995; 4: 353-387.
13. Dahlén GH: Atherosclerosis 1994; 108: 111-126.
14. Berglund L: Curr Opin Lipidol 1995; 6: 48-56.
15. Maher VMG, Brown BG: Curr Opin Lipidol 1995; 6: 229-235.
16. Krempler F, Kostner GM, Bolzano K, Sandhofer F: J Clin Invest 1980; 65: 1483-1490.
17. Rader DJ, Cain W, Ikewaki K, Talley G, Zech LA, Usher D, Brewer HB Jr: J Clin Invest 1994; 93: 2758-2763.
18. Hwang SB, J. Lipid Mediators, 1990: 2: 123-158.
19. Saunders RN, Handley DA. Ann Rev Pharmacol Toxicol 1987; 27: 237-255.
20. Weber KH, Heuer HO. Med Res Rev 1989; 9: 181-218.
21. Goodman & Gilman's The pharmacological basis of therapeutics. Eds. Hardman JG, Limbird LE, Molinoff PB, Ruddon RW, Goodman Gilman A. 9th edition 1996. McGraw-Hill, New York.
22. Komecki E, Ehrlich Y, Lenox RH. Science 1984; 226: 1454-1456.
23. Gurakar A, Hoeg JM, Kostner G, Papadopoulos NM, Brewer HB. Atherosclerosis 1985; 57: 293-301
24. Carlson LA, Hamsten A, Asplund A. J Intern Med 1989; 226: 271-276
25. Kaptein A, de Wit ECM, Princen HMG. Arterioscler Thromb. 1993;13:1505-1514.
26. Princen HMG, Huijsmans CMG, Kuipers F, Vonk RJ, Kempen HJM. J Clin Invest 1986; 78: 1064-1071
27. Havekes LM, Verboom H, de Wit E, Yap SH, Princen HMG. Hepatology 1986; 6: 1356-1360
28. Kooistra T, Bosma PJ, Tons HAM, van den Berg AP, Meijer P, Princen HMG. Thromb Haemostas 1989; 62: 723-728
29. Makino K, Abe A, Maeda S, Noma A, Kawada M, Takenaka O. Atherosclerosis. 1989; 78:81-85.
30. Azrolan N, Gavish D, Breslow JL. J Biol Chem. 1991; 266:13866-13872.
31. Kaptein A, Roodenburg L, Princen HMG. Biochem J. 1991; 278:557-564.
32. Kaptein A, de Wit ECM, Princen HMG. Arterioscler Thromb. 1994; 14:780-789
33. Chomczynski P, Sacchi N. Anal Biochem 1987; 162: 156-159.
34. McLean JW, Tomlinson JE, Kuang WJ, Eaton DL, Chen EY, Fless GM, Scanu AM, Lawn RM. Nature 1987; 300: 132-137.

## Claims

1. Use of an azepine derivative having formula 1, 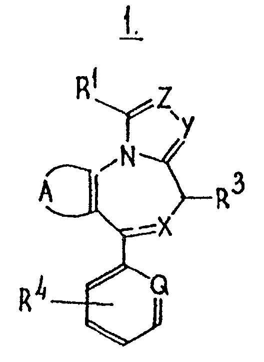 wherein
A is a group -CH=C(R²)-CH=CH-, -S-C(R²)=CH-, -CH=C(R²)-S- or a group having formula: 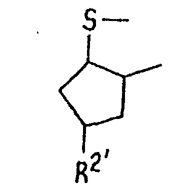 so as to form an optionally substituted benzo or thieno ring,
Q is a nitrogen atom (-N=) or an optionally substituted carbon atom (-CR⁴=),
X is a nitrogen, sulphur or oxygen atom, the neighbouring bond optionally being a double bond if X is nitrogen,
Y is a nitrogen atom (=N-) or an optionally substituted carbon atom (=CR-, wherein R = hydrogen, methyl, ethyl or (m)ethoxycarbonyl),
Z is a nitrogen atom (-N=) or an optionally methyl-substituted carbon atom (-CR'=, wherein R' = hydrogen, methyl or hydroxymethyl),
at least one of Y and Z being a nitrogen atom,
R¹ is hydrogen, halogen or C₁-C₆ alkyl, cycloalkyl(alkyl) or alkenyl, trifluoromethyl, hydroxymethyl or aminomethyl,
R² is hydrogen, halogen, trifluoromethyl, nitro, C₁-C₃ alkyl or a group -CH₂-CH₂R^{2'},
R^{2'} is optionally substituted phenyl, C₁-C₃ alkoxycarbonyl, or aminomethyl or carbamoyl the nitrogen atom of which may be substituted by one or two C₁-C₃ alkyl groups or may be part of a pyrrolidino, piperidino, or morpholino ring,
R³ is hydrogen, hydroxyl, methyl or carboxyl, and
R⁴ is hydrogen, halogen, hydroxyl, methyl or methoxy,
for preparing a medicament for treating or preventing atherosclerotic conditions by decreasing the synthesis of apolipoprotein(a) and/or decreasing the plasma level of lipoprotein(a).

2. Use according to claim 1, wherein R¹ is hydrogen, halogen or methyl and/or R³ is hydrogen or methyl.

3. Use according to claim 1 or 2, wherein R² is hydrogen or halogen.

4. Use according to any one of claims 1-3, wherein Q is -CR⁴= and R⁴ is hydrogen, 2-chloro or 3-hydroxy.

5. Use according to any one of claims 1-4, wherein X is nitrogen, the neighbouring bond being a double bond, and/or Y and Z are both nitrogen.

6. Use of a benzoazepine derivative having formula 1a, 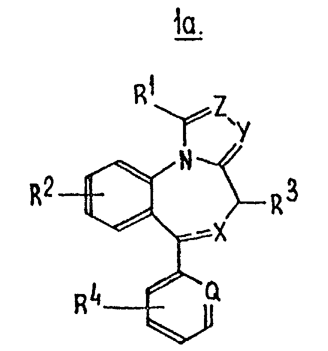 wherein
Q is a nitrogen atom (-N=) or an optionally substituted carbon atom (-CR⁴=),
X is a nitrogen, sulphur or oxygen atom, the neighbouring bond optionally a double bond if X is nitrogen,
Y is a nitrogen atom (=N-) or an optionally substituted carbon atom (=CR-, wherein R = hydrogen, methyl, ethyl or (m)ethoxycarbonyl),
Z is a nitrogen atom (-N=) or an optionally methyl-substituted carbon atom (-CR'=, wherein R' = hydrogen, methyl or hydroxymethyl),
at least one of Y and Z being a nitrogen atom,
R¹ is hydrogen, halogen or C₁-C₆ alkyl, cycloalkyl(alkyl) or alkenyl, trifluoromethyl, hydroxymethyl or aminomethyl,
R² is hydrogen, halogen, trifluoromethyl, nitro, or C₁-C₃ alkyl,
R³ is hydrogen, hydroxyl, methyl or carboxyl, and
R⁴ is hydrogen, halogen, hydroxyl, methyl or methoxy,
for preparing a medicament for treating or preventing atherosclerotic conditions by decreasing the synthesis of apolipoprotein(a) and/or decreasing the plasma level of lipoprotein(a) and/or increasing the synthesis of apolipoprotein A1 and/or increasing the plasma level of high-density lipoprotein.

7. Use according to claim 6, wherein R¹ is hydrogen, halogen or methyl, and/or R² is hydrogen or halogen and/or R³ is hydrogen or methyl, and/or Q is -CR⁴=, R⁴ being hydrogen, 2-chloro or 3-hydroxy and/or X, Y and Z are nitrogen, the neighbouring bond of X being a double bond.

8. Use of a substituted tetrahydrofuran phospholipid analogue having formula 2, 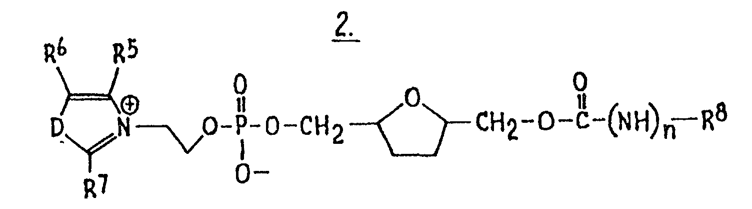 wherein D is -CR⁷=CR⁷- or -S-,
R⁵ and R⁶ are independently hydrogen, halogen, hydroxy, C₁-C₃ alkyl or C₁-C₃ alkoxy, or together are -(CH₂)₄-, -CH=CH-CH=CH-, -CR⁷=CR⁷-CR⁷=CR⁷- or -CH₂OCH₂-, each R⁷ is independently hydrogen, halogen, hydroxy, C₁-C₃ alkyl or C₁-C₃ alkoxy, preferably none or one of the symbols R⁷ being other than hydrogen,
R⁸ is C₈-C₂₀ alkyl, alkenyl, cycloalkyl-alkyl or arylalkyl, and
n is 0 or 1,
in particular the analogue denoted as PAF-antagonist SRI 63-441,
for preparing a medicament for treating or preventing atherosclerotic conditions by decreasing the synthesis of apolipoprotein(a) and/or decreasing the plasma level of lipoprotein(a) and/or increasing the synthesis of apolipoprotein A1 and/or increasing the plasma level of high-density lipoprotein.

9. Use of a diphenyl-tetrahydrofuran and diphenyl-dioxolane derivative, substituted on the phenyls by one or more hydroxy, C₁-C₃ alkyl, C₁-C₃ alkoxy, methylenedioxy groups and/or halogen atoms, in particular cis-2,4-bis(3,4,5-trimethoxyphenyl)-dioxolane, for preparing a medicament for treating or preventing atherosclerotic conditions by decreasing the synthesis of apolipoprotein(a) and/or decreasing the plasma level of lipoprotein(a) and/or increasing the synthesis of apolipoprotein A1 and/or increasing the plasma level of high-density lipoprotein.

10. Use according to any one of claims 1-9, wherein the compound is used in an amount between 0.01 and 30 mg per kg body weight per day.

## Patentansprüche

1. Verwendung eines Azepinderivats mit der Formel 1: 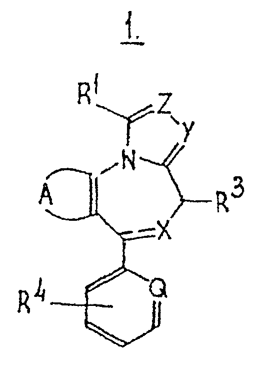 in der
A eine Gruppe -CH=C(R²)-CH=CH-, -S-C(R²)=CH-, -CH=C(R²)-Soder eine Gruppe mit der Formel: 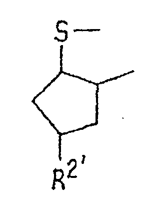 ist, um so einen gegebenenfalls substituierten Benzo- oder Thienoring zu bilden,
Q ein Stickstoffatom (-N=) oder ein gegebenenfalls substituiertes Kohlenstoffatom (-CR⁴=) ist,
X ein Stickstoff-, Schwefel- oder Sauerstoffatom ist, wobei die Nachbarbindung gegebenenfalls eine Doppelbindung ist, wenn X Stickstoff ist,
Y ein Stickstoffatom (=N-) oder ein gegebenenfalls substituiertes Kohlenstoffatom ist (=CR-, wobei R = Wasserstoff, Methyl, Ethyl oder (M)ethoxycarbonyl ist),
Z ein Stickstoffatom (-N=) oder ein gegebenenfalls methylsubstituiertes Kohlenstoffatom ist (-CR'=, wobei R' = Wasserstoff, Methyl oder Hydroxymethyl ist),
mindestens eines von Y und Z ein Stickstoffatom ist,
R¹ Wasserstoff, Halogen oder C₁- bis C₆-Alkyl, Cycloalkyl-(alkyl) oder Alkenyl, Trifluormethyl, Hydroxymethyl oder Aminomethyl ist,
R² Wasserstoff, Halogen, Trifluormethyl, Nitro, C₁- bis C₃-Alkyl oder eine Gruppe -CH₂-CH₂R^{2'} ist, wobei R^{2'} gegebenenfalls substituiertes Phenyl, C₁- bis C₃-Alkoxylcarbonyl oder Aminomethyl oder Carbamoyl ist, wobei das Stickstoffatom davon durch ein oder zwei C₁- bis C₃-Alkylgruppen substituiert sein kann oder Teil eines Pyrrolidino-, Piperidinooder Morpholinorings ist,
R³ Wasserstoff, Hydroxyl, Methyl oder Carboxyl ist und
R⁴ Wasserstoff, Halogen, Hydroxyl, Methyl oder Methoxy ist,
zur Herstellung eines Medikaments zur Behandlung oder Verhinderung von atherosklerotischen Erkrankungen durch Verringerung der Synthese von Apolipoproptein (a) und/oder Verringerung des Plasmaspiegels an Lipoprotein (a).

2. Verwendung nach Anspruch 1, bei der R¹ Wasserstoff, Halogen oder Methyl ist und/oder R³ Wasserstoff oder Methyl ist.

3. Verwendung nach Anspruch 1 oder 2, bei der R² Wasserstoff oder Halogen ist.

4. Verwendung nach einem der Ansprüche 1 bis 3, bei der Q -CR⁴= ist und R⁴ Wasserstoff, 2-Chlor- oder 3-Hydroxy- ist.

5. Verwendung nach einem der Ansprüche 1 bis 4, bei der X Stickstoff ist, die benachbarte Bindung eine Doppelbindung ist und/oder Y und Z beide Stickstoff sind.

6. Verwendung eines Benzoazepinderivats mit der Formel 1a 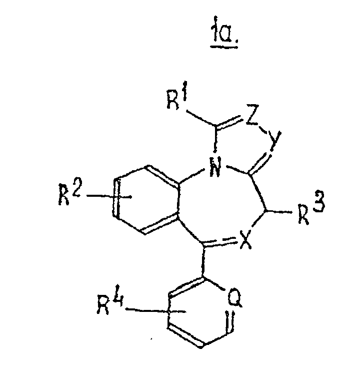 in der
Q ein Stickstoffatom (-N=) oder ein gegebenenfalls substituiertes Kohlenstoffatom (-CR⁴=) ist,
X ein Stickstoff-, Schwefel- oder Sauerstoffatom ist, wobei die benachbarte Bindung gegebenenfalls eine Doppelbindung ist, wenn X Stickstoff ist,
Y ein Stickstoffatom (=N-) oder ein gegebenenfalls substituiertes Kohlenstoffatom ist (=CR-, wobei R = Wasserstoff, Methyl, Ethyl oder (M)ethoxycarbonyl ist),
Z ein Stickstoffatom (-N=) oder ein gegebenenfalls methylsubstituiertes Kohlenstoffatom ist (-CR'=, wobei R' = Wasserstoff, Methyl oder Hydroxymethyl ist),
mindestens eines von Y und Z ein Stickstoffatom ist,
R¹ Wasserstoff, Halogen oder C₁- bis C₆-Alkyl, Cycloalkyl-(alkyl) oder Alkenyl, Trifluormethyl, Hydroxymethyl oder Aminomethyl ist,
R² Wasserstoff, Halogen, Trifluormethyl, Nitro oder C₁- bis C₃-Alkyl ist,
R³ Wasserstoff, Hydroxyl, Methyl oder Carboxyl ist und
R⁴ Wasserstoff, Halogen, Hydroxyl, Methyl oder Methoxy ist,
zur Herstellung eines Medikaments zur Behandlung oder Verhinderung von atherosklerotischen Erkrankungen durch Verringerung der Synthese von Apolipoproptein (a) und/oder Verringerung des Plasmaspiegels von Lipoprotein (a) und/oder Steigerung der Synthese von Apolipoprotein A1 und/oder Steigerung des Plasmaspiegels von Lipoprotein hoher Dichte.

7. Verwendung nach Anspruch 6, bei der R¹ Wasserstoff, Halogen oder Methyl ist und/oder R² Wasserstoff oder Halogen ist und/oder R³ Wasserstoff oder Methyl ist und/oder Q -CR⁴= ist, wobei R⁴ Wasserstoff, 2-Chlor oder 3-Hydroxy ist, und/oder X, Y und Z Stickstoff sind, wobei die benachbarte Bindung von X eine Doppelbindung ist.

8. Verwendung eines substituierten Tetrahydrofuranphospholipidanalogen mit der Formel 2 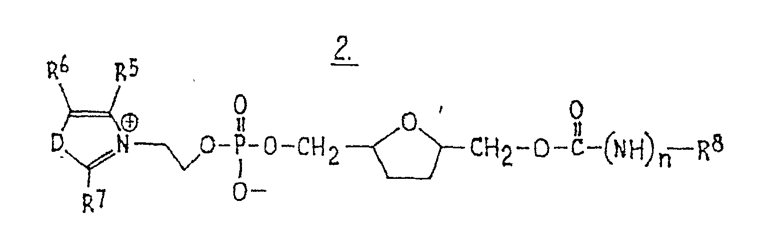 in der D -CR⁷=CH⁷- oder -S- ist,
R⁵ und R⁶ jeweils unabhängig Wasserstoff, Halogen, Hydroxy, C₁- bis C₃-Alkyl oder C₁- bis C₃-Alkoxy sind oder zusammen -(CH₂)₄-, -CH=CH-CH=CH-, -CR⁷=CR⁷-CR⁷=CR⁷- oder -CH₂OCH₂- sind, wobei jedes R⁷ jeweils unabhängig Wasserstoff, Halogen, Hydroxy, C₁- bis C₃-Alkyl oder C₁- bis C₃-Alkoxy ist, wobei vorzugsweise keines oder eines der Symbole R⁷ etwas anderes als Wasserstoff ist,
R⁸ C₈- bis C₂₀-Alkyl, Alkenyl, Cycloalkyl-alkyl oder Arylalkyl ist und
n 0 oder 1 ist,
insbesondere des Analogen, das als PAF-Antagonist SRI 63-441 bezeichnet ist,
zur Herstellung eines Medikaments zur Behandlung oder Verhinderung von atherosklerotischen Erkrankungen durch Verringerung der Synthese von Apolipoprotein (a) und/oder Verringerung des Plasmaspiegels von Lipoprotein (a) und/oder Steigerung der Synthese von Apolipoprotein A1 und/oder Steigerung des Plasmaspiegels von Lipoproptein hoher Dichte.

9. Verwendung eines Diphenyl-Tetrahydrofuran- und Diphenyl-Dioxolan-Derivats, das an den Phenylringen durch eine oder mehrere Hydroxy-, C₁- bis C₃-Alkyl-, C₁- bis C₃-Alkoxy-, Methylendioxygruppen und/oder Halogenatome substituiert ist, insbesondere cis-2,4-Bis(3,4,5-trimethoxyphenyl)dioxolan, zur Herstellung eines Medikaments zur Behandlung oder Verhinderung von atherosklerotischen Erkrankungen durch Verringerung der Synthese von Apolipoprotein (a) und/oder Verringerung des Plasmaspiegels von Lipoprotein (a) und/oder Steigerung der Synthese von Apolipoprotein A1 und/oder Steigerung des Plasmaspiegels an Lipoprotein hoher Dichte.

10. Verwendung nach einem der Ansprüche 1 bis 9, bei der die Verbindung in einer Menge von 0,01 bis 30 mg pro kg Körpergewicht pro Tag verwendet wird.

## Revendications

1. Utilisation d'un dérivé d'azépine ayant la formule 1, 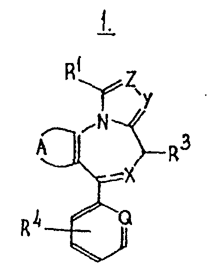 où
A est un groupe -CH=C(R²)-CH=CH-, -S-C(R²)=CH-, -CH=C(R²)-S- ou un groupe ayant la formule suivante : 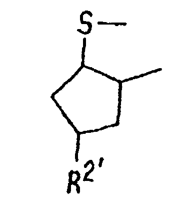 de manière à former un anneau benzo ou thiéno substitué de manière facultative,
Q est un atome d'azote (-N=) ou un atome de carbone substitué de manière facultative (-CR⁴=),
X est un atome d'azote, de souffre ou d'oxygène, la liaison voisine étant de manière facultative une double liaison si X est un azote,
Y est un atome d'azote (=N-) ou un atome de carbone substitué de manière facultative (=CR-, où R = hydrogène, méthyle, éthyle ou (m)éthoxycarbonyle),
Z est un atome d'azote (-N=) ou un atome de carbone méthyle-substitué de manière facultative (-CR'=, où R' = hydrogène, méthyle ou hydroxyméthyle),
au moins un élément parmi Y et Z étant un atome d'azote,
R¹ est un hydrogène, un halogène ou alkyle C₁-C₆, cycloalkyl(alkyle) ou alkényle, trifluorométhyle, hydroxyméthyle ou aminométhyle,
R² est un hydrogène, un halogène, un trifluorométhyle, un groupe nitro, un alkyle C₁-C₃ ou un groupe -CH₂-CH₂R^{2'},
R^{2'} et un phényle substitué de manière facultative, un alkoxycarbonyle C₁-C₃, ou un aminométhyle ou un carbamoyle dont l'atome d'hydrogène peut être substitué par un ou deux groupes alkyle C₁-C₃ ou peut être une partie d'un anneau pyrrolidino, pipéridino ou morpholino,
R³ est un hydrogène, un hydroxyle, un méthyle ou un carboxyle, et
R⁴ est un hydrogène, un halogène, un hydroxyle, un méthyle ou un méthoxy, pour préparer un médicament destiné à traiter ou prévenir des conditions d'athérosclérose en diminuant la synthèse d'apolipoprotéine (a) et/ou en diminuant le niveau de plasma d'une lipoprotéine (a).

2. Utilisation selon la revendication 1, dans laquelle R¹ est un hydrogène, un halogène ou un méthyle et/ou R³ est un hydrogène ou un méthyle.

3. Utilisation selon la revendication 1 ou 2, dans laquelle R² est un hydrogène ou un halogène.

4. Utilisation selon l'une quelconque des revendications 1 à 3, où Q est -CR⁴= et R⁴ est un hydrogène, un groupe 2-chloro ou 3-hydroxy.

5. Utilisation selon l'une quelconque des revendications 1 à 4, dans laquelle X est un azote, la liaison voisine étant une double liaison, et/ou Y et Z sont tous deux de l'azote.

6. Utilisation d'un dérivé de benzoazépine ayant la formule 1a, 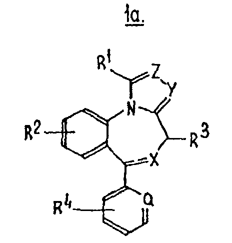 où
Q est un atome d'azote (-N=) ou un atome de carbone substitué de manière facultative (-CR⁴=),
X est un atome d'azote, de souffre ou d'oxygène, la liaison voisine étant de manière facultative une double liaison si X est un azote,
Y est un atome d'azote (=N-) ou un atome de carbone substitué de manière facultative (=CR-, où R = hydrogène, méthyle, éthyle ou (m)éthoxycarbonyle),
Z est un atome d'azote (-N=) ou un atome de carbone méthyle-substitué de manière facultative (-CR'=, où R' = hydrogène, méthyle ou hydroxyméthyle),
au moins un élément parmi Y et Z étant un atome d'azote,
R¹ est un hydrogène, un halogène ou alkyle C₁-C₆, cycloalkyl(alkyle) ou alkényle, trifluorométhyle, hydroxyméthyle ou aminométhyle,
R² est un hydrogène, un halogène, un trifluorométhyle, un groupe nitro, ou un alkyle C₁-C₃,
R³ est un hydrogène, un hydroxyle, un méthyle ou un carboxyle, et
R⁴ est un hydrogène, un halogène, un hydroxyle, un méthyle ou un méthoxy, pour préparer un médicament destiné à traiter ou prévenir des conditions d'athérosclérose en diminuant la synthèse d'apolipoprotéine (a) et/ou en diminuant le niveau de plasma d'une lipoprotéine (a) et/ou en augmentant la synthèse de l'apolipoprotéine A1 et/ou en augmentant le niveau de plasma d'une lipoprotéine à haute densité.

7. Utilisation selon la revendication 6, dans laquelle R¹ est un hydrogène, un halogène ou un méthyle, et/ou R² est un hydrogène ou un halogène et/ou R³ est un hydrogène ou un méthyle, et/ou Q est -CR⁴=, R⁴ étant de l'hydrogène, un groupe 2-chloro ou 3-hydroxy et/ou X, Y et Z sont un azote, la liaison voisine de X étant une double liaison.

8. Utilisation d'un analogue phospholipidique de tétrahydrofuranne substitué ayant la formule 2, 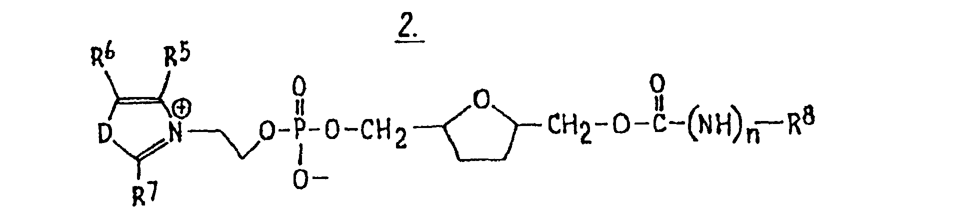 où D est -CR⁷=CR⁷- ou -S-,
R⁵ et R⁶ sont indépendamment un hydrogène, un halogène, un hydroxy, un alkyle C₁-C₃ ou un alkoxy C₁-C₃, ou ensemble sont -(CH₂)₄-, -CH=CH-CH=CH-, -CR⁷=CR⁷-CR⁷=CR⁷- ou -CH₂OCH₂-, chaque R⁷ est indépendamment un hydrogène, un halogène, un hydroxy, un alkyle C₁-C₃ ou un alkoxy C₁-C₃, de préférence aucun ou un des symboles R⁷ n'étant pas de l'hydrogène,
R⁸ est un alkyle C₈-C₂₀, un alkényle, un cyloalkyle-alkyle ou un arylalkyle, et n est égal à 0 ou 1,
en particulier un analogue désigné l'antagoniste SRI 63-441 de PAF,
pour préparer un médicament destiné à traiter ou prévenir des conditions d'athérosclérose en diminuant la synthèse d'une apolipoprotéine (a) et/ou en diminuant le niveau de plasma d'une lipoprotéine (a) et/ou en augmentant la synthèse de l'apolipoprotéine A1 et/ou en augmentant le niveau de plasma d'une lipoprotéine à haute densité.

9. Utilisation d'un dérivé de diphényle-tétrahydrofuranne et diphényle-dioxolane, substitué sur les phényles par un ou plusieurs groupes hydroxy, alkyle C₁-C₃, alkoxy C₁-C₃, méthylènedioxy et/ou atomes d'halogène, en particulier cis-2,4-bis(3,4,5-triméthoxyphényle)-dioxolane, pour préparer un médicament destiné à traiter ou prévenir des conditions d'athérosclérose en diminuant la synthèse d'une apolipoprotéine (a) et/ou en diminuant le niveau de plasma d'une lipoprotéine (a) et/ou en augmentant la synthèse d'une apolipoprotéine A1 et/ou en augmentant le niveau de plasma d'une lipoprotéine à haute densité.

10. Utilisation selon l'une quelconque des revendications 1 à 9, dans laquelle le composé est utilisé selon une quantité comprise entre 0,01 et 30 mg par kg de poids corporel par jour.
